# EUROPÄISCHE PATENTSCHRIFT

(11) **EP 0 528 120 B1**
(45) Veröffentlichungstag und Bekanntmachung des Hinweises auf die Patenterteilung: **17.05.1995**
(21) Anmeldenummer: 92109159.1
(22) Anmeldetag: 30.05.1992
(51) Int. Cl.: A61M 5/178, A61M 5/28, B65D 51/24

(54) **Spritze für medizinische Zwecke**
Syringe for medical purposes
Seringue pour des buts médicaux

(30) Priorität: 21.08.1991 DE 4127650
(43) Veröffentlichungstag der Anmeldung: 24.02.1993
(73) Patentinhaber: Arzneimittel GmbH Apotheker Vetter & Co. Ravensburg, D-88212 Ravensburg (DE)
(72) Erfinder: Vetter, Helmut, W-7980 Ravensburg (DE); Geprägs, Peter, W-7987 Weingarten (DE)
(74) Vertreter: Dziewior, Joachim, Dipl.-Phys. Dr.

(56) Entgegenhaltungen:
- EP-A- 0 006 032
- WO-A-87/06141
- DE-A- 3 736 343
- FR-A- 2 294 104

## Beschreibung

Die Erfindung betrifft eine Spritze für medizinische Zwecke, mit einem Spritzenzylinder und darin verschiebbaren Spritzenkolben und mit einem am nadelseitigen Ende des Spritzenzylinders axial aufgesetzten, im Rastsitz gehaltenen Nadelansatzstück, das mit einem hülsenförmigen Anschlußteil das mit einem Außenbund versehene nadelseitige Ende des Spritzenzylinders umgreift und an der Innenmantelfläche des Anschlußteils einen Ringvorsprung trägt, der in eine halsförmige Querschnittsverengung zwischen dem Außenbund und dem Spritzenzylinder greift.

Derartige, allgemein bekannte Spritzen, die mit einer lyophilisierten Substanz als Ein- oder Doppelkammerspritze befüllt in den Verkehr kommen, werden zunächst mit dem in gelöster Form vorliegenden Wirkstoff befüllt, sodann der Lyophilisierung unterzogen und schließlich noch im Lyophilisator geschlossen.

Der Erfindung liegt die Aufgabe zugrunde, eine Spritze der eingangs genannten Art so auszubilden, daß einerseits der Lyophilisierungsvorgang schnell ablaufen kann, insbesondere also ein ausreichend großer Öffnungsquerschnitt für den Durchtritt des entweichenden Lösungsmittels zur Verfügung steht, andererseits die Spritze unmittelbar im Anschluß an die Lyophilisierung noch in steriler Umgebung ohne aufwendige Hilfsmaßnahmen geschlossen werden kann.

Diese Aufgabe wird nach der Erfindung dadurch gelöst, daß das Nadelansatzstück einen koaxial im Anschlußteil angeordneten, zwischen sich und dem Anschlußteil einen Ringraum bildenden Verschlußstopfen trägt, dessen Außenkontur im wesentlichen der Innenkontur des Außenbundes und der Querschnittsverengung entspricht, daß ferner der Außenbund zwischen seiner Stirnfläche und der halsförmigen Querschnittsverengung eine Ringnut aufweist, in die der Ringvorsprung in einer ersten Raststellung des Nadelansatzstücks greift, in der der Ringraum mit dem Inneren des Spritzenzylinders über einen zwischen der Innenseite des Außenbunds und dem Verschlußstopfen gebildeten Ringspalt miteinander verbunden sind, und daß das Anschlußteil mit wenigstens einer von außen in den Ringraum sich erstreckenden Ausnehmung versehen ist.

Der durch die Erfindung erreichte Vorteil besteht im wesentlichen darin, daß sich die Spritze bereits in einem vormontierten Zustand befindet, so daß im Anschluß an die Lyophilisierung das bezüglich des Spritzenzylinders bereits zentrierte Nadelansatzstück lediglich vollständig auf das nadelseitige Ende des Spritzenzylinders aufgeschoben werden muß. Gleichwohl bildet der Ringspalt eine ausreichend große Öffnung, durch die das Lösungsmittel während der Lyophilisierung entweichen kann.

In bevorzugter Ausführungsform der Erfindung ist das Anschlußteil mit mehreren, über den Umfang gleichmäßig verteilt angeordneten Ausnehmungen versehen, die als axial verlaufende, sich bis zum freien Rand des Anschlußteils erstreckende Schlitze ausgebildet sind. Hierdurch steht für den Durchtritt des Lösungsmittels auch im Bereich des Anschlußteils ein ausreichend großer Querschnitt zur Verfügung, wobei durch die schlitzartige Ausbildung der Ausnehmungen darüber hinaus die Montage des Nadelansatzstücks am Spritzenzylinder vereinfacht wird.

Das Nadelansatzstück ist zur Aufnahme des Verschlußstopfens vorteilhafterweise mit einer sich vom Öffnungsrand aus konisch erweiternden Sackausnehmung versehen, in die der Verschlußstopfen mit einem der konischen Wandfläche angepaßten kegelstumpfförmigen Bodenteil eingesetzt ist. Dies ermöglicht eine einfache Montage des aus in der Regel weicherem Material gegenüber dem Nadelansatzstück bestehendem Verschlußstopfens, gewährleistet aber gleichzeitig einen festen Halt.

Der Verschlußstopfen kann grundsätzlich in einfachster Ausführungsform zylindrisch ausgebildet sein. Um eine bessere Abdichtung zu erreichen, weist der Verschlußstopfen in bevorzugter Ausführungsform der Erfindung in dem an seine freie Stirnseite angrenzenden Bereich einen eine Dichtlippe bildenden Ringwulst und einen sich daran über eine konisch verlaufende Fläche anschließenden zylindrischen Bereich von gegenüber dem Ringwulst größerem Durchmesser auf. Hierdurch entstehen in axialer Richtung des Spritzenzylinders mehrere Dichtbereiche, die in jedem Fall eine hochwertige Abdichtung gewährleisten.

Das Nadelansatzstück kann einen durch ein Tip-Cap geschlossenen Konus zum Ansetzen der Kanüle aufweisen, in den hinein sich der Verschlußstopfen mit einem Ansatzstück erstreckt.

Insbesondere dann, wenn sich die Ausnehmungen bis zum freien Rand des Anschlußteils erstrecken, empfiehlt es sich, daß das Nadelansatzstück durch einen das Anschlußteil umgreifenden Sicherungsring fixiert ist. Hierbei kann der Sicherungsring seinerseits Mittel aufweisen, die sein Entfernen erschweren oder unmöglich machen.

Um die Montage des Sicherungsrings zu vereinfachen, sieht die Erfindung weiter vor, daß der Sicherungsring an seinem zum Spritzenzylinder weisenden Rand ein Verbindungsglied zum Anschluß am nadelseitigen Ende des hülsenförmigen Anschlußteils aufweist. Hierdurch befindet sich der Sicherungsring in einer Position, aus der er durch axiale Verschiebung auf das Anschlußteil vollständig aufgesetzt werden kann. Hierbei ist es von Vorteil, wenn das Verbindungsglied von einer radial nach innen vorstehenden Randleiste gebildet ist, die in eine radial nach außen offene Ringnut am Anschlußteil vorsteht. Die Montage des Nadelansatzstücks sowie das Aufsetzen des Sicherungsrings auf das Anschlußteil kann dabei in einem einzigen Arbeitshub vorgenommen werden, wenn sicher gestellt ist, daß die zum Lösen des Ringvorsprungs aus der Ringnut des Außenbundes erforderliche Kraft geringer ist als diejenige, die zum Lösen des Verbindungsgliedes zwischen dem Sicherungsring und dem Anschlußteil notwendig ist. Im übrigen kann die am Verbindungsglied radial nach innen vorstehende Randleiste zugleich ein Sicherungselement bilden, das das Abziehen des auf das Anschlußteil aufgesetzten Sicherungsrings verhindert.

In einer vorteilhaften Fortbildung der Erfindung kann am Sicherungsring über einen als Sollbruchstelle ausgebildeten Anschlußsteg eine bei auf das Anschlußteil aufgeschobenem Sicherungsring den Konus für die Kanüle bzw. das darauf aufgesetzte Tip-Cap umschließende Sicherungskappe angeschlossen sein. Die Unversehrtheit des Anschlußstegs bzw. das Vorhandensein der Sicherungskappe stellt dann sicher, daß sich die Spritze noch in ihrem Originalzustand befindet. Die Sicherungskappe kann dabei mit einer stirnseitig angebrachten axialen Bohrung versehen sein, deren lichter Durchmesser kleiner als der Außendurchmesser des Tip-Cap ist. Diese Bohrung ermöglicht eine Sichtkontrolle beispielsweise auf Vorhandensein des Tip-Cap.

Der Spritzenzylinder kann schließlich an seinem dem Nadelansatzstück abgewandten Ende eine radial nach außen vorstehende Nase tragen, die in eine axial verlaufende Schlitzausnehmung der Fingerauflage greift. Hierdurch wird ein drehfester Anschluß des Nadelansatzstücks erreicht.

Im folgenden wird die Erfindung an in der Zeichnung dargestellten Ausführungsbeispielen näher erläutert; es zeigen:
- Fig. 1: in den Teilfiguren a) bis d) die Spritze in verschiedenen Stadien während des Befüllungsvorgangs,
- Fig. 2: eine der Fig. 1 entsprechende Darstellung einer zweiten Ausführungsform der Erfindung,
- Fig. 3: eine Detaildarstellung der Fig. 1a bis 1c,
- Fig. 4: eine Detaildarstellung der Fig. 2a bis 2c,
- Fig. 5: einen Querschnitt durch den Gegenstand nach Fig. 1a,
- Fig. 6: einen Querschnitt bzw. Längsschnitt durch die Fingerauflage der Spritze.

Die in der Zeichnung dargestellte Spritze für medizinische Zwecke besteht aus einem Spritzenzylinder 1, ferner zumindest einem darin verschiebbaren Spritzenkolben 2 und einem am nadelseitigen Ende des Spritzenzylinders 1 axial aufgesetzten Nadelansatzstück 3, das im Rastsitz gehalten ist. Das Nadelansatzstück 3 ist mit einem hülsenförmigen Anschlußteil 4 versehen, das den Außenbund 5 am nadelseitigen Ende des Spritzenzylinders 1 umgreift.

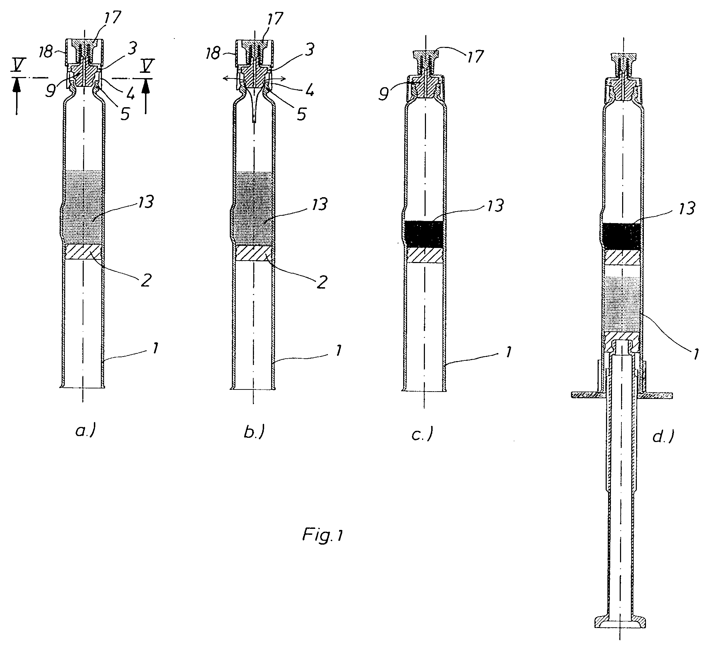

Das Nadelansatzstück 3 trägt an der Innenmantelfläche des Anschlußteils 4 einen Ringvorsprung 6, der in eine halsförmige Querschnittsverengung 7 zwischen dem Außenbund 5 und dem Spritzenzylinder 1 greift, wie dies jeweils aus der Teilfigur c) der Fig. 1 bis 4 ersichtlich ist.

Das Nadelansatzstück 3 trägt einen koaxial im Anschlußteil 4 angeordneten, zwischen sich und dem Anschlußteil 4 einen Ringraum 8 bildenden Verschlußstopfen 9. Die Außenkontur des Verschlußstopfens 9 entspricht im wesentlichen der Innenkontur des Außenbundes 5 und der Querschnittsverengung 7, so daß ein in axialer Richtung ausreichend langer Dichtbereich gegeben ist.

Der Außenbund 5 weist zwischen seiner Stirnfläche und der halsförmigen Querschnittsverengung 7 eine Ringnut 10 auf, in die der Ringvorsprung 6 in einer ersten Raststellung des Nadelansatzstücks 3 greift. In dieser ersten Raststellung sind der Ringraum 8 mit dem Inneren des Spritzenzylinders 1 über einen zwischen der Innenseite des Außenbunds 5 und dem Verschlußstopfen 9 gebildeten Ringspalt 11 miteinander verbunden. Schließlich ist das Anschlußteil 4 mit wenigstens einer von außen in den Ringraum 8 sich erstreckenden Ausnehmung 12 versehen.

In der jeweils in den Teilfiguren a) und b) dargestellten ersten Raststellung erfolgt die Lyophilisierung des in gelöster Form in den Spritzenzylinder 1 abgefüllten Wirkstoffs 13, wobei das Lösungsmittel über den Ringspalt 11, den Ringraum 8 und die Ausnehmungen 12 im Anschlußteil 4 entweichen kann.

Das Anschlußteil 4 ist mit mehreren, über den Umfang gleichmäßig verteilt angeordneten Ausnehmungen 12 versehen, die als axial verlaufende Schlitze ausgebildet sind. Diese Schlitze erstrecken sich bis zum freien Rand des Anschlußteils 4.

Das Nadelansatzstück 3 weist eine sich vom Öffnungsrand aus konisch erweiternde Sackausnehmung 14 auf, in die der Verschlußstopfen 9 mit einem kegelstumpfförmigen, der konischen Wandfläche angepaßten Bodenteil 15 eingesetzt ist.

Der Verschlußstopfen 9 weist in dem an seine freie Stirnseite angrenzenden Bereich einen eine Dichtlippe bildenden Ringwulst 9.1 auf. An den Ringwulst 9.1 schließt sich eine konisch verlaufende Fläche 9.2 und an diese ein zylindrischer Bereich 9.3 an, der gegenüber dem Ringwulst 9.1 einen größeren Durchmesser aufweist.

Das Nadelansatzstück 3 ist mit einem Konus 16 zum Aufsetzen der Kanüle versehen, der durch ein Tip-Cap 17 geschlossen ist. Der Verschlußstopfen 9 weist ein Ansatzstück 9.4 auf, das sich bis in den Konus 16 hinein erstreckt und eine zusätzliche Abdichtung gegenüber dem Tip-Cap 17 bewirkt.

Das vollständig auf den Außenbund 5 aufgeschobene Nadelansatzstück 3 ist, wie jeweils die Teilfigur c) der Fig. 1 bis 4 zeigt, durch einen das Anschlußteil 4 umgreifenden Sicherungsring 18 fixiert. Dieser Sicherungsring 18 verhindert ein erneutes Aufspreizen der zwischen den schlitzförmigen Ausnehmungen 12 gebildeten Lamellen 19, wobei der Sicherungsring 18 zusätzlich so ausgebildet sein kann, daß er aus seinem auf das Anschlußteil 4 aufgeschobenen Zustand nicht oder nur schwer entfernt werden kann.

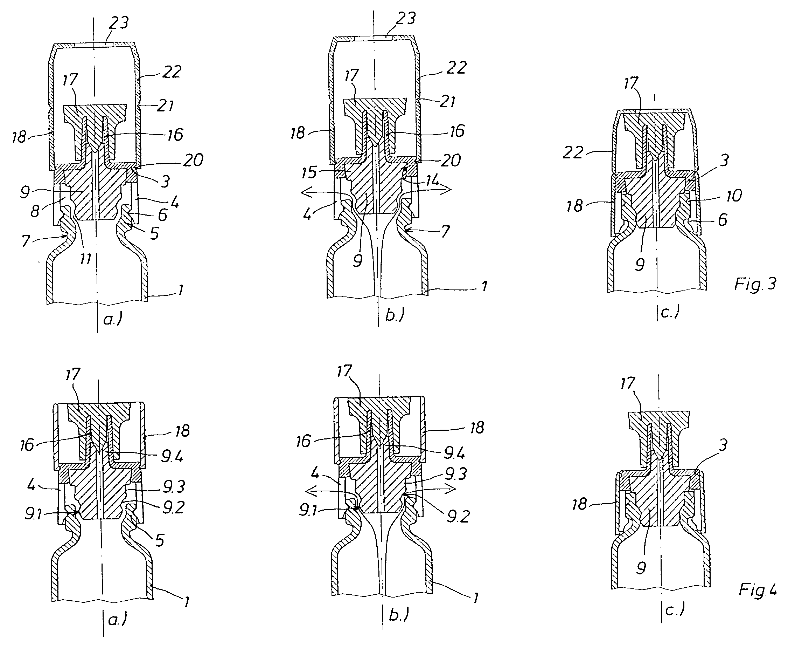

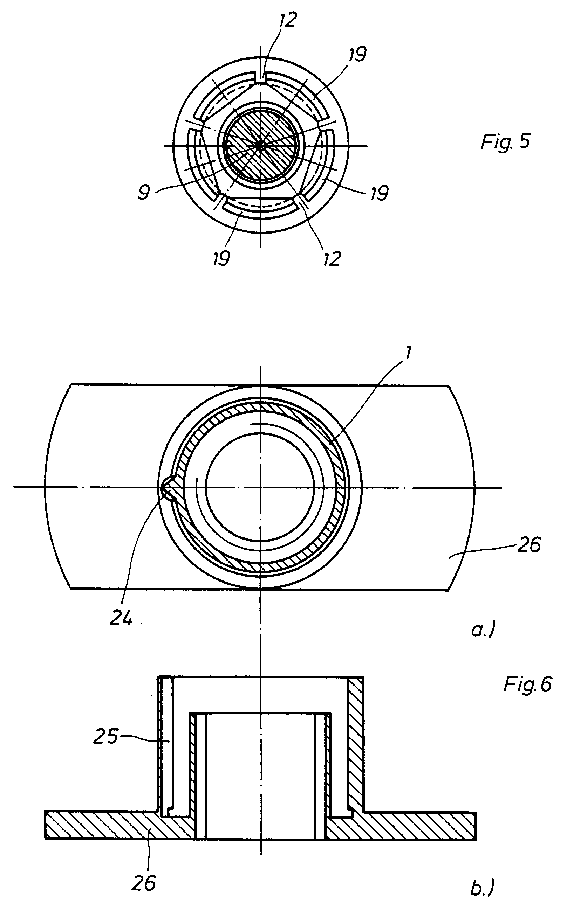

Vor dem Aufschieben auf das Anschlußteil 4 ist der Sicherungsring 18 an seinem zum Spritzenzylinder 1 weisenden Rand über ein Verbindungsglied am nadelseitigen Ende des hülsenförmigen Anschlußteils 4 angeschlossen. Dieses Verbindungsglied 20 ist von einer radial nach innen vorstehenden Randleiste gebildet, die in eine radial nach außen offene Ringnut am Anschlußteil 4 vorsteht, wie dies aus den Fig. 3 und 4 ersichtlich ist.

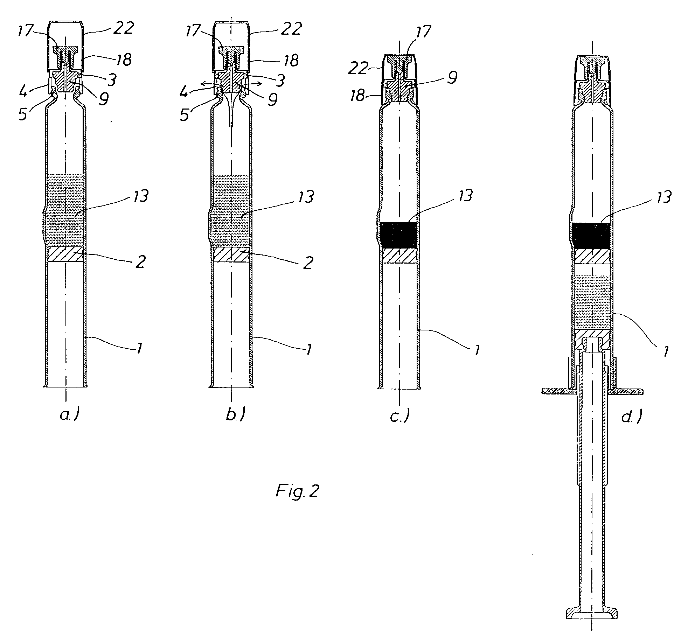

In der Ausführungsform nach den Fig. 2 und 4 ist am Sicherungsring 18 über einen als Sollbruchstelle ausgebildeten Anschlußsteg 21 eine Sicherungskappe 22 angeschlossen, die bei auf das Anschlußteil 4 aufgeschobenem Sicherungsring 18 den Konus 16 für die Kanüle bzw. das darauf aufgesetzte Tip-Cap 17 umschließt. Diese Sicherungskappe 22 ist stirnseitig mit einer axialen Bohrung 23 versehen, deren lichter Durchmesser kleiner als der Außendurchmesser des Tip-Cap 17 ist. Auf diese Weise kann durch die Bohrung eine Sichtkontrolle erfolgen, ohne daß die Möglichkeit besteht, das Tip-Cap 17 von dem Konus 16 abzuziehen. Hierdurch ist ein ohne weiteres nachprüfbarer Originalitätsverschluß gewährleistet.

Der Spritzenzylinder 1 trägt an seinem dem Nadelansatzstück 3 abgewandten Ende eine radial nach außen vorstehende Nase 24, die in eine axial verlaufende Schlitzausnehmung 25 der Fingerauflage 26 greift. Hierdurch ist eine drehfeste Montage der Fingerauflage 26 am Spritzenzylinder 1 gewährleistet.

## Patentansprüche

1. Spritze für medizinische Zwecke, mit einem Spritzenzylinder (1) und darin verschiebbarem Spritzenkolben (2) und einem am nadelseitigen Ende des Spritzenzylinders (1) axial aufgesetzten, im Rastsitz gehaltenen Nadelansatzstück (3), das mit einem hülsenförmigen Anschlußteil (4) das mit einem Außenbund (5) versehene nadelseitige Ende des Spritzenzylinders (1) umgreift und an der Innenmantelfläche des Anschlußteils einen Ringvorsprung (6) trägt, der in eine halsförmige Querschnittsverengung (7) zwischen dem Außenbund (5) und dem Spritzenzylinder (1) greift, dadurch gekennzeichnet, daß das Nadelansatzstück (3) einen koaxial im Anschlußteil (4) angeordneten, zwischen sich und dem Anschlußteil (4) einen Ringraum (8) bildenden Verschlußstopfen (9) trägt, dessen Außenkontur im wesentlichen der Innenkontur des Außenbundes (5) und der Querschnittsverengung (7) entspricht, daß ferner der Außenbund (5) zwischen seiner Stirnfläche und der halsförmigen Querschnittsverengung (7) eine Ringnut (10) aufweist, in die der Ringvorsprung (6) in einer ersten Raststellung des Nadelansatzstücks (3) greift, in der der Ringraum (8) und das Innere des Spritzenzylinders (1) über einen zwischen der Innenseite des Außenbundes und dem Verschlußstopfen (9) gebildeten Ringspalt (11) miteinander verbunden sind, und daß das Anschlußteil (4) mit wenigstens einer von außen in den Ringraum (8) sich erstreckenden Ausnehmung (12) versehen ist.

2. Spritze nach Anspruch 1, dadurch gekennzeichnet, daß das Anschlußteil (4) mit mehreren, über den Umfang gleichmäßig verteilt angeordneten Ausnehmungen (12) versehen ist, die als axial verlaufende, sich bis zum freien Rand des Anschlußteiles (4) erstreckende Schlitze ausgebildet sind.

3. Spritze nach Anspruch 1 oder 2, dadurch gekennzeichnet, daß das Nadelansatzstück (3) zur Aufnahme des Verschlußstopfens (9) mit einer sich vom Öffnungsrand aus konisch erweiternden Sackausnehmung (14) versehen ist, in die der Verschlußstopfen (9) mit einem der konischen Wandfläche angepaßten, kegelstumpfförmigen Bodenteil (15) eingesetzt ist.

4. Spritze nach einem der Ansprüche 1 bis 3, dadurch gekennzeichnet, daß der Verschlußstopfen (9) in dem an seine freie Stirnseite angrenzenden Bereich einen eine Dichtlippe bildenden Ringwulst (9.1) und einen sich daran über eine konisch verlaufende Fläche (9.2) anschließenden zylindrischen Bereich (9.3) von gegenüber dem Ringwulst (9.1) größerem Durchmesser aufweist.

5. Spritze nach einem der Ansprüche 1 bis 4, dadurch gekennzeichnet, daß das Nadelansatzstück (3) einen durch ein Tip-Cap (17) geschlossenen Konus (16) zum Aufsetzen der Kanüle aufweist, in den hinein sich der Verschlußstopfen (9) mit einem Ansatzstück (9.4) erstreckt.

6. Spritze nach einem der Ansprüche 1 bis 5, dadurch gekennzeichnet, daß das Nadelansatzstück (3) durch einen das Anschlußteil (4) umgreifenden Sicherungsring (18) fixiert ist.

7. Spritze nach Anspruch 6, dadurch gekennzeichnet, daß der Sicherungsring (18) an seinem zum Spritzenzylinder weisenden Rand ein Verbindungsglied (20) zum Anschluß am nadelseitigen Ende des hülsenförmigen Anschlußteils (4) aufweist.

8. Spritze nach Anspruch 7, dadurch gekennzeichnet, daß das Verbindungsglied (20) von einer radial nach innen vorstehenden Randleiste gebildet ist, die in eine radial nach außen offene Ringnut am Anschlußteil (4) vorsteht.

9. Spritze nach einem der Ansprüche 6 bis 8, dadurch gekennzeichnet, daß am Sicherungsring (18) über einen als Sollbruchstelle ausgebildeten Anschlußsteg (21) eine bei auf das Anschlußteil (4) aufgeschobenen Sicherungsring (18) den Konus (16) für die Kanäle bzw. das darauf aufgesetzte Tip-Cap (17) umschließende Sicherungskappe (22) angeschlossen ist.

10. Spritze nach Anspruch 9, dadurch gekennzeichnet, daß die Sicherungskappe (22) mit einer stirnseitig angebrachten axialen Bohrung (23) versehen ist, deren lichter Durchmesser kleiner als der Außendurchmesser des Tip-Cap (17) ist.

11. Spritze nach einem der Ansprüche 1 bis 10, dadurch gekennzeichnet, daß der Spritzenzylinder (1) an seinem dem Nadelansatzstück (3) abgewandten Ende eine radial nach außen vorstehende Nase (24) trägt, die in eine axial verlaufende Schlitzausnehmung (25) der Fingerauflage (26) greift.

## Claims

1. A syringe for medical purposes comprising a syringe cylinder (1) and a syringe plunger (2) displaceable in the syringe cylinder, and a needle attachment portion (3) which is axially fitted on the syringe cylinder (1) at the needle end thereof and which is held by a latching fit and which with a sleeve-like connecting portion (4) embraces the needle end of the syringe cylinder (1) which is provided with an external collar (5), the needle attachment portion at the internal peripheral surface of the connecting portion carrying an annular projection (6) which engages into a neck-shaped reduction in cross-section (7) between the external collar (5) and the syringe cylinder (1), characterised in that the needle attachment portion (3) carries a closure plug (9) which is arranged coaxially in the connecting portion (4) and which forms between itself and the connecting portion (4) an annular space (8), the external contour of the closure plug substantially corresponding to the internal contour of the external collar (5) and the reduction in cross-section (7), that in addition between its end face and the neck-shaped reduction in cross-section (7) the external collar (5) has an annular groove (10) into which the annular projection (6) engages m a first latching position of the needle attachment portion (3) in which the annular space (8) and the interior of the syringe cylinder (1) are connected together by way of an annular gap (11) formed between the inside of the external collar and the closure plug (9), and that the connecting portion (4) is provided with at least one opening (12) which extends from the outside into the annular space (8).

2. A syringe according to claim 1 characterised in that the connecting portion (4) is provided with a plurality of openings (12) which are arranged uniformly distributed around the periphery and which are in the form of axially extending slots which extend as far as the free edge of the connecting portion (4).

3. A syringe according to claim 1 or claim 2 characterised in that for receiving the closure plug (9) the needle attachment portion (3) is provided with a blind opening (14) which enlarges conically from the orifice edge and into which the closure plug (9) is fitted with a frustoconical end portion (15) which is adapted to the conical wall surface.

4. A syringe according to one of claims 1 to 3 characterised in that in the region adjoining its free end the closure plug (9) has an annular ridge (9.1) forming a sealing lip and a cylindrical region (9.3) of larger diameter than the annular ridge (9.1), said cylindrical region joining the annular ridge by way of a conically extending surface (9.2).

5. A syringe according to one of claims 1 to 4 characterised in that the needle attachment portion (3) has a cone (16) for fitting the cannula thereon, the cone being closed by a tip cap (17) and the closure plug (9) extending into the cone with an attachment portion (9.4).

6. A syringe according to one of claims 1 to 5 characterised in that the needle attachment portion (3) is fixed by a security ring (18) which embraces the connecting portion (4).

7. A syringe according to claim 6 characterised in that at its edge which faces towards the syringe cylinder the security ring (18) has a connecting member (20) for connection to the needle end of the sleeve-shaped connecting portion (4).

8. A syringe according to claim 7 characterised in that the connecting member (20) is formed by a radially inwardly projecting edge bar which projects into a radially outwardly open annular groove on the connecting portion (4).

9. A syringe according to one of claims 6 to 8 characterised in that a security cap (22) is connected to the security ring (18) by way of a connecting limb (21) in the form of a desired-rupture location, which security cap encloses the cone (16) for the cannula or the tip cap (17) fitted thereon when the security ring (18) is pushed on to the connecting portion (9).

10. A syringe according to claim 9 characterised in that the security cap (22) is provided with an axial bore (23) which is disposed at its end and the internal diameter of which is smaller than the outside diameter of the tip cap (17).

11. A syringe according to one of claims 1 to 10 characterised in that at its end remote from the needle attachment portion (3) the syringe cylinder (1) carries a radially outwardly projecting nose (24) which engages into an axially extending slot opening (25) in the finger support (26).

## Revendications

1. Seringue destinée à des fins médicales, comportant un cylindre de seringue (1) et un piston de seringue (2) pouvant coulisser dans le cylindre, et un embout d'aiguille (3), monté de façon axiale sur l'extrémité du cylindre de seringue qui se trouve du côté de l'aiguille, et maintenu fixe, ledit embout d'aiguille enveloppant, à l'aide d'un élément de raccordement (4) en forme de douille, l'extrémité du cylindre de seringue située du côté de l'aiguille et pourvue d'une collerette externe, et portant sur la surface de paroi interne de l'élément de raccordement un anneau en saillie (6) qui pénètre dans un rétrécissement transversal (7) entre le collerette (5) et le cylindre de seringue (1), caractérisé en ce que l'embout d'aiguille (3) porte un bouchon d'obturation (9) disposé de façon coaxiale dans l'élément de raccordement (4) et formant entre celui-ci et l'embout d'aiguille (3) un espace annulaire (8), dont le contour externe correspond pratiquement au contour interne du rétrécissement transversal (7), en ce qu'en outre la collerette (5) présente entre se surface frontale et le rétrécissement transversal (7), une rainure (10) dans laquelle pénètre l'anneau en saillie (6) lors d'une première position d'arrêt de l'embout d'aiguille, dans laquelle l'espace annulaire (8) et l'intérieur du cylindre de seringue (1) sont reliés par l'intermédiaire d'un passage annulaire (11) formé entre l'intérieur de la collerette et le bouchon d'obturation (9), et en ce que l'élément de raccordement (4) est pourvu d'au moins un creux (12) s'étendant de l'extérieur vers l'espace annulaire (8).

2. Seringue selon la revendication 1, caractérisée en ce que l'élément de raccordement (4) est pourvu de plusieurs creux (12) régulièrement répartie sur son pourtour, lesdits creux ayant la forme de fentes orientées axialement et s'étendant jusqu'au bord libre de l'élément de raccordement (4).

3. Seringue selon la revendication 1 ou 2, caractérisée en ce que l'embout d'aiguille (3) est pourvu, pour la réception du bouchon d'obturation (9), d'un creux en forme de poche (14) s'élargissant de façon conique à partir du bord d'ouverture, dans laquelle le bouchon d'obturation (9) est positionné avec une partie de base (15) en forme de cône tronqué, adaptée à la paroi conique.

4. Seringue selon l'une des revendications 1 à 3, caractérisée en ce que le bouchon d'obturation (9) présente dans la zone voisine de sa face libre frontale, un anneau (9.1) formant une lèvre d'étancheité, et une zone cylindrique (9.3) ayant un diamètre supérieur à celui de l'anneau (9.1) et reliée à l'anneau par l'intermédiaire d'une surface cylindrique (9.2).

5. Seringue selon l'une des revendications 1 à 4, caractérisée en ce que l'embout d'aiguille (3) présente un cône (16) fermé par un bouchon d'extrémité (17), prévu pour l'application de la canule, et dans lequel le bouchon d'obturation s'étend avec un élément de raccordement (9.4).

6. Seringue selon l'une des revendications 1 à 5, caractérisée en ce que l'embout d'aiguille (3) est fixé par un anneau de sécurité (18) entourant l'élément de raccordement (4).

7. Seringue selon la revendication 6, caractérisée en ce que l'anneau de sécurité (18) comporte, sur son bord orienté vers le cylindre de seringue, un élément de liaison (20) pour la connexion à l'extrémité de l'élément de raccordement (4) en forme de douille qui est située du côté de l'aiguille.

8. Seringue selon la revendication 7, caractérisée en ce que l'élément de liaison (20) est formé à partir d'un rebord s'étendant radialement vers l'intérieur, pénétrant dans une rainure de l'élément de raccordement (4), ouverte radialement vers l'extérieur.

9. Seringue selon l'une des revendications 6 à 8, caractérisée en ce qu'une capsule de sécurité (22) qui entoure le cône (16) pour les canules ou, respectivement, le bouchon d'extrémité (17) lorsque l'anneau de sécurité (18) est repoussé sur l'élément de raccordement (4), est reliée à l'anneau de sécurité (18) par une nervure (21) réalisée sous la forme d'une zone à rupture programmée.

10. Seringue selon la revendication 9, caractérisée en ce que la capsule de sécurité (22) est pourvue d'un trou d'alésage axial (23), disposé sur la face frontale, dont le diamètre d'ouverture est inférieur au diamètre externe du bouchon d'extrémité (17).

11. Seringue selon l'une des revendications 1 à 10, caractérisée en ce que le cylindre de seringue (1) comporte, à son extrémité opposée par rapport à l'embout d'aiguille (3), un talon (24) s'étendant radialement vers l'extérieur et pénétrant dans une ouverture en forme de fente (25) des appuis (26) pour les doigts.
